(19) Europäisches Patentamt
European Patent Office
Office européen des brevets

(11) Veröffentlichungsnummer: **0 438 795 A1**

# (12) EUROPÄISCHE PATENTANMELDUNG

(21) Anmeldenummer: **90125638.8**

(22) Anmeldetag: **28.12.90**

(51) Int. Cl.⁵: **C07D 213/81**, C07D 213/82, A61K 31/44

(30) Priorität: **16.01.90 DE 4001002**

(43) Veröffentlichungstag der Anmeldung:
**31.07.91 Patentblatt 91/31**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Baader, Ekkehard, Dr.**
**Amselweg 14**
**W-6240 Königstein/Taunus(DE)**

## (54) Pyridin-2,4- und 2,5-dicarbonsäuredi(nitroxyalkyl) amide, Verfahren zu deren Herstellung sowie deren Verwendung.

(57) Die Erfindung betrifft Pyridin-2,4- und -2,5-dicarbonsäuredi-(nitroxyalkyl)amide der Formel I

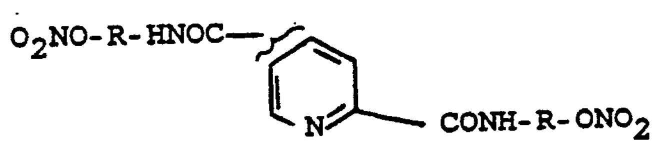

(I)

worin R die angegebenen Bedeutungen hat. Die erfindungsgemäßen Verbindungen hemmen die Enzyme Prolin- und Lysinhydroxylase und können dementsprechend als Fibrosuppressiva und Immunsuppressiva eingesetzt werden.

EP 0 438 795 A1

# PYRIDIN-2,4-UND 2,5-DICARBONSÄUREDI-(NITROXYALKYL)AMIDE, VERFAHREN ZU DEREN HERSTELLUNG SOWIE DEREN VERWENDUNG

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie $\alpha,\alpha'$ -Dipyridyl zu einer Hemmung der $C1_q$-Biosynthese von Makrophagen führt (W. Müller et al., FEBS Lett. 90 (1978), 218; Immunbiology 155 (1978), 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen.

So wird in der DE-A 37 03 959 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In den deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamidvorgeschlagen. Die deutsche Patentanmeldung P 3924093.2 schlägt neue N,N'-Bis(alkoxyalkyl)-pyridin-2,4-dicarbonsäurediamide vor.

Sowohl Pyridin-2,4- und -2,5-dicarbonsäurediamid (Hirakata et al., J. pharm. Soc. Japan 77 (1957) 219 und Häring et al., Helv. 37 (1954) 147, 153) als auch Pyridin-2,4 und -2,5-dicarbonsäuredihydrazid (Itai et al., Bl. nation. hyg. Labor. Tokyo, 74 (1956) 115, 117 und Shinohara et al., Chem. High Polymers Japan, 15 (1958) 839) sind bereits als Tuberkulosemittel bekannt.

In der JP 53/28175 (78/28175) werden N,N'-bis(2-nitrooxyethyl)pyridin-2,4-und -2,5-dicarbonsäurediamide als Substanzen mit vasodilatorischer Wirkung beschrieben.

Überraschend wurde nun gefunden, daß Pyridin-2,4- und 2,5-dicarbonsäuredi-(nitroxyalkyl)amide der Formel I

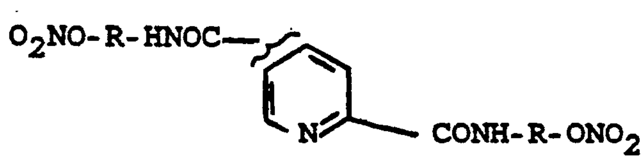

(I)

worin

R $C_1$-$C_4$-Alkandiyl bedeutet

sowie die physiologisch verträglichen Salze, die Lysin- und Prolinhydroxylase im Tiermodell wirksam inhibieren.

Die Erfindung betrifft dementsprechend a) die Verwendung von Verbindungen der Formel I

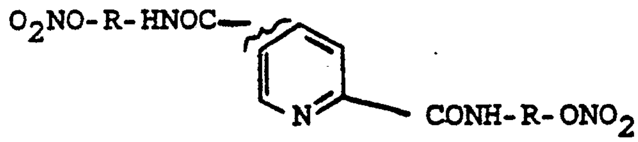

(I)

worin

R $C_1$-$C_4$-Alkandiyl bedeutet sowie die physiologisch verträglichen Salze, zur Herstellung eines Prolin- und Lysinhydroxylase hemmenden Arzneimittels.

Weiterhin betrifft die Erfindung b) die Verbindungen der Formel I,

worin

R Methylen, Propylen oder Butylen bedeutet sowie die physiologisch verträglichen Salze, zur Verwendung als Arzneimittel.

Weiterhin betrifft die Erfindung c) die Verbindungen der Formel I,

worin

R Methylen, Propylen oder Butylen bedeutet sowie deren physiologisch verträglichen Salze.

Insbesondere betrifft die Erfindung die Verbindungen der Formel I gemäß a), b) und c) zur Anwendung als Fibrosuppressiva und Immunsuppressiva sowie zur Inhibierung der Prolin- und Lysinhydroxylase und zur Beeinflussung des Stoffwechsels von Kollagen und kollagenähnlichen Stoffen bzw. der Biosynthese von $C1_q$.

Alle genannten Alkylreste mit mehr als 2-C-Atomen können sowohl geradkettig als auch verzweigt sein.

Weiterhin betrifft die Erfindung ein Verfahren zur Herstellung von Verbindungen der Formel I, das dadurch gekennzeichnet ist, daß man eine Verbindung der Formel II

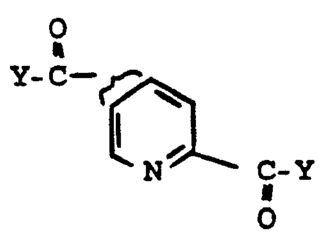

(II)

mit einer Verbindung der Formel III

$H_2N$-R-$ONO_2$ (III)

umsetzt, wobei R die zu Formel I angegebenen Bedeutung hat und Y Halogen, Hydroxy oder $C_1$-$C_4$-Alkoxy ist oder zusammen mit der Carbonylgruppe einen Aktivester oder ein gemischtes Anhydrid bildet oder daß man eine Verbindung der Formel IV

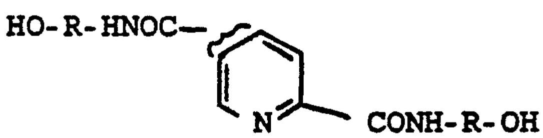

(IV)

worin

R wie oben definiert ist, nitriert und anschließend die Reaktionsprodukte gegebenenfalls in ihre physiologisch verträglichen Salze überführt.

Im folgenden wird die Herstellung von Verbindungen gemäß Formel I und die Herstellung der dafür benötigten Ausgangssubstanzen - sofern sie nicht käuflich sind - näher beschrieben.

Die Herstellung der erfindungsgemäßen Verbindungen gelingt am einfachsten dadurch, daß die beiden Komponenten, das Pyridin-Derivat gemäß Formel (II) und das Amin gemäß Formel (III) in äquimolaren

Mengen oder bis zu einem etwa 5-fachen Überschuß an III zusammengegeben werden und bei Temperaturen zwischen -30 bis 150° C, bevorzugt bei 20 bis 100 °C bis zur Beendigung der Reaktion umgesetzt werden. Die Beendigung der Reaktion läßt sich beispielsweise mittels Dünnschichtchromatographie bestimmen. Eine Variante dieses Verfahrens besteht darin, daß man in einem geeigneten Lösungsmittel, wie Diäthyläther oder Dimethoxyäthan oder Tetrahydrofuran, chlorierten Kohlenwasserstoffen wie Methylenchlorid, Chloroform, Tri- oder Tetrachloräthylen, Benzol, Toluol oder auch polaren Lösungsmitteln wie Dimethylformamid, Aceton, Alkoholen wie Methanol oder Ethanol oder Dimethylsulfoxid arbeitet. Auch hier kann ein Überschuß von Amin gemäß Formel (III) der bis zur etwa 5-fachen Mengen betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130° C besonders bevorzugt sind.

Ebenso kann die Umsetzung über ein gemisches Anhydrid wie Chlorameisensäureäthylester oder über einen aktivierten Ester wie Paranitrophenylester (Y = $ClCH_2$ -COO oder $NO_2$-$C_6H_4$-O) erfolgen. Entsprechende Methoden sind in der Literatur beschrieben.

Gegebenenfalls kann die Umsetzung auch in Gegenwart von Basen erfolgen. Als zusätzliche Basen kommen beispielsweise Carbonate oder Hydrogencarbonate wie Natrium- oder Kaliumcarbonat oder Natrium- oder Kaliumhydrogencarbonat, oder tertiäre Amine, wie Triäthylamin, Tributylamin, Äthyldiisopropylamin oder heterocyclische Amine wie N-Alkylmorpholin, Pyridin, Chinolin oder Dialkylaniline in Betracht.

Eine Variante zur Darstellung der Verbindungen der Formel I besteht darin, daß man die entsprechenden Hydroxyalkyldiamide der Pyridin-2,4- bzw. -2,5-dicarbonsäure (IV) nitriert. Dabei werden die entsprechenden Hydroxyalkyldiamide bei Reaktionstemperaturen von -20° C bis +10° C, bevorzugt bei -10° C bis -5° C mit konzentrierter Salpetersäure versetzt. Die Reaktionszeit beträgt hierbei 10-240 Min., bevorzugt 20-90 Min. Anschließend wird das Reaktionsprodukt gegebenenfalls neutralisiert.

Gegebenenfalls kann die Aufarbeitung der Produkte beispielsweise durch Extraktion oder durch Chromatographie z.B. über Kieselgel erfolgen. Das isolierte Produkt kann umkristallisiert und gegebenenfalls mit einer geeigneten Säure zu einem physiologisch verträglichen Salz umgesetzt werden. Als geeignete Säuren kommen beispielsweise in Betracht:

Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion, Bernstein-, Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (III) können, soweit sie nicht käuflich sind, nach literaturbekannten Verfahren synthetisiert werden.

Die Ausgangsverbindungen der Formel (II) erhält man beispielsweise durch Umsetzung von Pyridin-2,4- oder 2,5-dicarbonsäure zu dem entsprechenden Pyridin-2,4- oder 2,5-dicarbonsäurehalogenid, bevorzugt -chlorid (nach literaturbekannten Verfahren), vorzugsweise in Gegenwart eines Katalysators wie Dimethylformamid. Dieses Säurehalogenid kann dann beispielsweise entweder mit einem geeigneten Alkohol, z.B. Paranitrobenzylalkohol zu dem entsprechenden Aktivester, oder aber mit niederen Alkoholen wie Methanol oder Ethanol zu den entsprechenden Estern umgesetzt werden. Ebenso kann die Pyridin-2,4- oder 2,5-dicarbonsäure auch zunächst unter Zusatz einer geeigneten Carbonsäure oder eines Carbonsäureesters wie Chlorameisensäureäthylester in ein gemischtes Anhydrid überführt werden, welches dann mit dem Aminen (III) zu den erfindungsgemäßen Produkten umgesetzt wird. Eine entsprechende Methode ist ebenfalls in der Literatur beschrieben.

Die Ausgangsverbindungen der Formel IV erhält man beispielsweise durch Umsetzung entsprechender N,N'-Bis(alkoxyalkyl)pyridin-2,4- bzw. -2,5-dicarbonsäurediamide, bevorzugt dem Bis(methoxyalkyl)diamid nach literaturbekannten Verfahren, beispielsweise mit Bortribromid. Die Herstellung der Bis(alkoxyalkyl)-diamide ist bekannt und beispielsweise beschrieben in der DE-A 3 703 959. Hierbei wird eine reaktionsfähige Pyridindicarbonsäure, beispielsweise das Pyridin-dicarbonsäurechlorid mit einem Alkoxyalkylamin umgesetzt.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum und Immunsuppressivum.

Auf Grund dieser pharmakologischen Eigenschaften sind die erfindungsgemäßen Verbindungen zur Behandlung von Störungen des Stoffwechsels von Kollagen und kollagenähnlichen Stoffen, bzw. zur Behandlung von Störungen der Biosynthese von C1q geeignet.

Die Erfindung betrifft daher weiter die Anwendung der erfindungsgemäßen Verbindungen der Formel I sowie deren physiologisch verträglichen Salze bei der Behandlung der oben genannten Stoffwechselstörungen.

Die Verbindungen können entweder allein oder mit physiologisch verträglichen Hilfs- oder Trägerstoffen vermischt als Arzneimittel angewandt werden. Sie können zu diesem Zweck oral in Dosen von 0,01 - 25,0 mg/kg/Tag, vorzugsweise 0,01 - 5,0 mg/kg/Tag oder parenteral in Dosen von 0,001 - 5 mg/kg/Tag, vorzugsweise 0,001 - 2,5 mg/kg/Tag, inbesondere 0,005 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75% beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral oder rektal appliziert werden.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen.

Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

**Vorstufe 1:**

Pyridin-2,4-dicarbonsäure-bis-N,N'-(methoxyethyl)amid

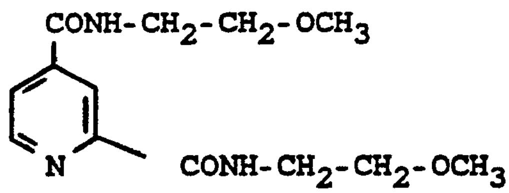

3 g Pyridin-2,4-dicarbonsäure werden in 50 ml Toluol und 1 ml DMF vorgelegt und zur Lösung 2,7 ml Thionylchlorid zugetropft. Es wird solange erhitzt, bis keine Gasentwicklung mehr zu beobachten ist (ca. 2,5 h). Es wird abgekühlt, 5 ml Toluol abdestilliert und zur Lösung 4,6 ml 2-Methoxyethylamin und 5 ml Triethylamin zugetropft. Nach Rühren der Lösung bei Raumtemperatur für 4 h wird eingedampft, der Rückstand mit Wasser aufgenommen und 4 x mit Methylenchlorid extrahiert. Die vereinigten organischen Phasen werden über Magnesiumsulfat getrocknet und eingedampft. Das Rohprodukt wird mit Kieselgel (Lösungsmittel Ethylacetat) chromatographiert.

Fp.: 42 - 44 °C

$^1$H-NMR ($CDCl_3$) :

δ = 1,2 (3H, tr); 3,3-3,8 (12H, qu. und m); 7,9 (1H, m); 8,4-8,5 (1H, m); 8,7-8,8 (1H, m);

**Vorstufe 2:**

Pyridin-2,4-dicarbonsäure-bis-N,N'-(2-hydroxyethyl)amid

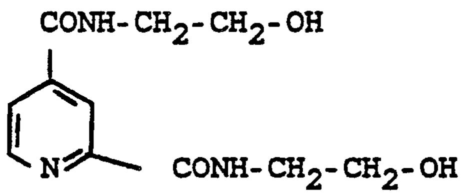

0,5 g Pyridin-2,4-dicarbonsäure-bis-N,N'-(2-methoxyethyl)-amid (aus Vorstufe 1) werden in 10 ml Dichlormethan gelöst und bei -78 °C Bortribromid (11 ml, 1 molare Lösung in Dichlormethan) zugetropft. Nach beendeter Zugabe auf Raumtemperatur kommen lassen und 3 Stunden nachrühren. Auf 100 ml gesattigte Bicarbonat-Lösung gießen und 3 x mit Ethylacetat extrahieren. Die vereinigten organischen Lösungsmittel werden mit Magnesiumsulfat getrocknet und eingedampft. Das Rohprodukt wird an Kieselgel chromatographiert.
$^1$H-NMR ($CDCl_3$) :
δ = 1,5-2,2 (4H, m); 3,4 (4H, m); 3,6 (4H, m); 7,9-8,0 (1H, m); 8,4-8,5 (1H, m); 8,7-8,8 (1H, m)

**Beispiel 1**

Pyridin-2,4-dicarbonsäure-N,N'-di[2-(nitroxy)-ethyl]amid

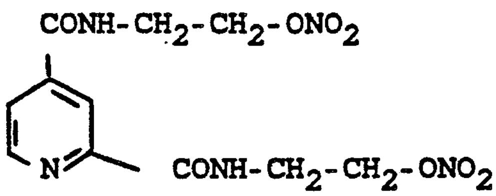

1 g Pyridin-2,4-dicarbonsäure-di-(2-hydroxyethyl)-amid (Vorstufe 2) werden bei -10 °C bis -5 °C in 5ml konzentrierte Salpetersäure gegeben. Man läßt auf 2 °C erwärmen und 40 Minuten nachrühren. Es wird auf Eis/Wasser gegossen und mit Natriumcarbonat neutralisiert. Die Lösung wird dreimal mit Dichlormethan extrahiert, die org. Phasen über Magnesiumsulfat getrocknet und eingedampft. Der Rückstand kristallisiert aus Ether aus.
Ausbeute: 750 mg
Fp.: 85 - 88 °C

**Patentansprüche**

1. Verwendung von Pyridin-2,4- und -2,5-dicarbonsäuredi-(nitroxyalkyl)amiden der Formel I

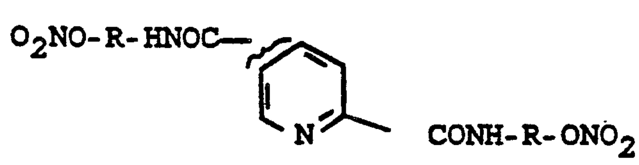

(I)

worin
R lineares oder verzweigtes $C_1$-$C_4$-Alkandiyl bedeutet
sowie die physiologisch verträglichen Salze, zur Herstellung von Prolin- und Lysinhydroxylase hemmenden Arzneimitteln.

2. Verwendung nach Anspruch 1, dadurch gekennzeichnet, daß R Ethylen oder Propylen bedeutet.

3. Verbindungen der Formel I

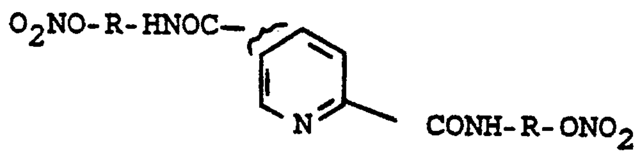

(I)

worin
R Methylen, Propylen oder Butylen bedeutet,
sowie die physiologisch verträglichen Salze.

4. Verbindungen der Formel I nach Anspruch 3, dadurch gekennzeichnet, daß R Ethylen oder n-Propylen bedeutet.

5. Verbindungen nach Anspruch 3 oder 4 zur Anwendung als Arzneimittel.

6. Verfahren zur Herstellung von Verbindungen der Formel I nach Anspruch 3, dadurch gekennzeichnet, daß man eine Verbindung der Formel II

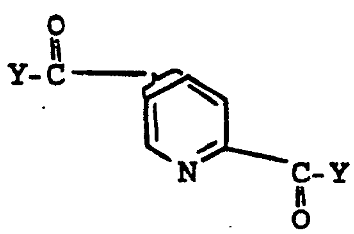

(II)

mit einer Verbindung der Formel III

$H_2N\text{-}R\text{-}ONO_2$ (III)

umsetzt, wobei R die in Anspruch 3 angegebenen Bedeutungen hat und Y Halogen, Hydroxy oder $C_1$-$C_4$-Alkoxy ist oder zusammen mit der Carbonylgruppe einen Aktivester oder ein gemischtes Anhydrid bildet oder daß man eine Verbindung der Formel IV

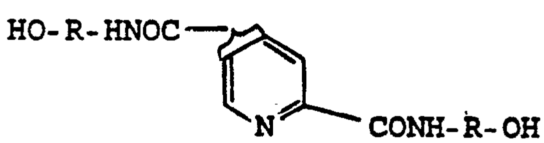

IV

worin R wie in Anspruch 3 definiert ist, nitriert und anschließend die Reaktionsprodukte gegebenenfalls in ihre physiologisch verträglichen Salze überführt.

7. Verbindungen nach Anspruch 3 oder 4 zur Inhibierung der Prolin- und Lysinhydroxylase.

8. Verbindungen nach einem oder mehreren der Ansprüche 1 bis 4 zur Anwendung als Fibrosuppressiva und Immunsuppressiva.

9. Arzneimittel, enthaltend eine Verbindung der Formel I gemäß Anspruch 3 oder 4 mit verträglichen pharmazeutischen Trägern.

**10.** Verwendung von Verbindungen der Formel I oder deren Salze gemäß einem oder mehreren der Ansprüche 1 bis 4 zur Beeinflussung des Stoffwechsels von Kollagen und kollagenähnlichen Stoffen bzw. der Biosynthese von $C1_q$.

**11.** Verwendung von Verbindungen der Formel I oder deren Salze gemäß einem oder mehreren der Ansprüche 1 bis 4 zur Behandlung von Störungen des Stoffwechsels von Kollagen und kollagenähnlichen Stoffen bzw. der Biosynthese von $C1_q$.

**12.** Verfahren zur Herstellung von Arzneimitteln zur Beeinflussung des Stoffwechsels von Kollagen und kollagenähnlichen Stoffen bzw. der Biosynthese von $C1_q$,dadurch gekennzeichnet, daß man dem Arzneimittel eine Verbindung der Formel I oder deren Salze gemäß einem oder mehreren der Ansprüche 1 bis 4 hinzugibt.

**Patentansprüche für folgenden Vertragsstaat: ES**

**1.** Verwendung von Pyridin-2,4- und -2,5-dicarbonsäuredi-(nitroxyalkyl)amiden der Formel I

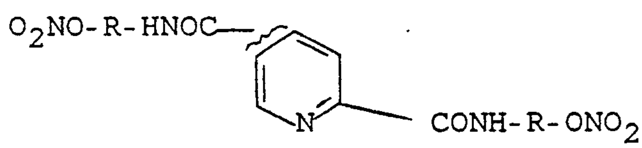

(I)

worin
R lineares oder verzweigtes $C_1$-$C_4$-Alkandiyl bedeutet
sowie die physiologisch verträglichen Salze, zur Herstellung von Prolin- und Lysinhydroxylase hemmenden Arzneimitteln.

**2.** Verwendung nach Anspruch 1, dadurch gekennzeichnet, daß
R Ethylen oder Propylen bedeutet.

**3.** Verfahren zur Herstellung einer Verbindung der Formel I

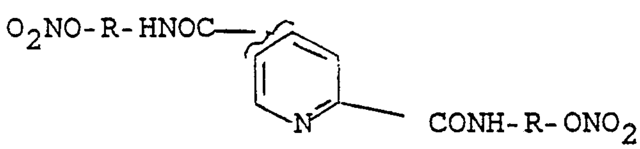

(I)

worin
R Methylen, Propylen oder Butylen bedeutet,
sowie deren physiologisch verträglichen Salze, dadurch gekennzeichnet, daß man eine Verbindung der Formel II

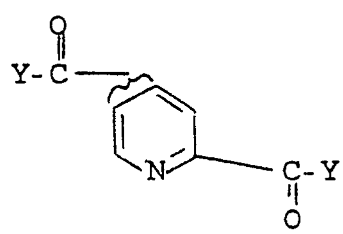

(II)

mit einer Verbindung der Formel III

$H_2N$-R-$ONO_2$ (III)

umsetzt, wobei R die oben angegebenen Bedeutungen hat und Y Halogen, Hydroxy oder $C_1$-$C_4$-Alkoxy ist oder zusammen mit der Carbonylgruppe einen Aktivester oder ein gemischtes Anhydrid bildet oder, daß man eine Verbindung der Formel IV

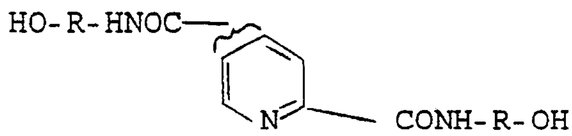

(IV)

worin R wie oben definiert ist, nitriert und anschließend die Reaktionsprodukte gegebenenfalls in ihre physiologisch verträglichen Salze überführt.

4. Verfahren nach Anspruch 3, dadurch gekennzeichnet, daß R Methylen oder n-Propylen bedeutet.

5. Arzneimittel enthaltend eine Verbindung der Formel I gemäß Ansprüchen 1 bis 4 mit verträglichen pharmazeutischen Trägern.

6. Arzneimittel gemäß Anspruch 5 zur Inhibierung der Prolin- und Lysinhydroxylase.

7. Arzneimittel gemäß Anspruch 5 oder 6, zur Anwendung als Fibrosuppressiva und Immunsuppressiva.

8. Verwendung von Verbindungen der Formel I oder deren Salze gemäß einem oder mehreren der Ansprüche 1 bis 4 zur Beeinflussung des Stoffwechsels von Kollagen und kollagenähnlichen Stoffen bzw. der Biosynthese von $C1_q$.

9. Verwendung von Verbindungen der Formel I oder deren Salze gemäß einem oder mehreren der Ansprüche 1 bis 4 zur Behandlung von Störungen des Stoffwechsels von Kollagen und kollagenähnlichen Stoffen bzw. der Biosynthese von $C1_q$.

10. Verfahren zur Herstellung von Arzneimitteln zur Beeinflussung des Stoffwechsels von Kollagen und kollagenähnlichen Stoffen bzw. der Biosynthese von $C1_q$,dadurch gekennzeichnet, daß man dem Arzneimittel eine Verbindung der Formel I oder deren Salze gemäß einem oder mehreren der Ansprüche 1 bis 4 hinzugibt.

Europäisches Patentamt

# EUROPÄISCHER TEILRECHERCHENBERICHT,
der nach Regel 45 des Europäischen Patentübereinkommens für das weitere Verfahren als europäischer Recherchenbericht gilt

Nummer der Anmeldung
EP 90125638

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0278453 (HOECHST AG)<br>* Ansprüche 1,5,8-10,13 *; & DE-A-3703959 (Kat. D) | 1-9,12 | C07D213/81<br>C07D213/82<br>A61K31/44 |
| A | EP-A-0176741 (HOECHST AG)<br>* Ansprüche 1,5-10 *;<br>& DE-A-3432094 (Kat. D) | 1,5,<br>7-9,12 | |
| A | CHEMICAL ABSTRACTS<br>Band 89, Nr. 5, 31. Juli 1978, Seite 604, Zusammenfassung Nr. 43132z, Columbus, Ohio, US; & JP - A - 7828175 (Kat. D)<br>* Zusammenfassung * | 3-6 | |
| A | DE-A-2714713 (CHUGAI SEIYAKU K.K.)<br>* Seite 10, Zeilen 7-9; Ansprüche 1,2, 10,11; Figur 14 * | 3-6 | |

RECHERCHIERTE SACHGEBIETE (Int. Cl.5)

A61K31/00
C07D213/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9,12
Unvollständig recherchierte Patentansprüche: –
Nicht recherchierte Patentansprüche: 10,11
Grund für die Beschränkung der Recherche:

Artikel 52(4) EPÜ

| Recherchenort | Abschlußdatum der Recherche | Prüfer |
|---|---|---|
| Berlin | 24.04.1991 | C. HASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82